# EUROPEAN PATENT SPECIFICATION

(11) **EP 2 692 359 B1**
(45) Date of publication and mention of the grant of the patent: **07.06.2017**
(21) Application number: 12765137.0
(22) Date of filing: 28.03.2012
(51) Int. Cl.: A61K 39/395, A61P 35/00, A61P 37/04

(54) **CANCER IMMUNOPOTIENTING AGENT CONTAINING RANKL ANTAGONIST**
KREBSIMMUNITÄTSVERSTÄRKER MIT EINEM RANKL-ANTAGONISTEN
POTENTIALISATEUR D'IMMUNITÉ ANTICANCÉREUSE CONTENANT UN ANTAGONISTE DE RANKL

(30) Priority: 31.03.2011 JP 2011079167
(43) Date of publication of application: 05.02.2014
(73) Proprietor: Oriental Yeast Co., Ltd., Tokyo 174-8505 (JP)
(72) Inventor: AKIYAMA, Taishin, Tokyo 108-8639 (JP); YANAI, Hiromi, Tokyo 108-8639 (JP); AKIYAMA, Nobuko, Tokyo 108-8639 (JP); YASUDA, Hisataka, Tokyo 174-8505 (JP)
(74) Representative: Hoffmann Eitle
(86) International application number: PCT/JP2012/059130
(87) International publication number: WO 2012/133914

(56) References cited:
- WO-A1-2010/022120
- WO-A1-2011/017294
- WO-A2-2008/088594
- JP-A- 2005 515 159
- JP-A- 2005 515 752
- MARIE-LIESSE ASSELIN-LABAT ET AL: "Control of mammary stem cell function by steroid hormone signalling", NATURE, vol. 465, no. 7299, 11 April 2010 (2010-04-11), pages 798-802, XP055152179, ISSN: 0028-0836, DOI: 10.1038/nature09027
- YASUSHI KUBOTA ET AL.: 'Role of Osteoclasts Targeting Agents, Bisphosphonates and RANKL Inhibitor, Against Cancer Stem Cells' SAISHIN IGAKU vol. 66, no. 3, 10 March 2011, pages 19 - 24, XP008171090
- SHUNJI TAKAHASHI: 'Hone Ten'i ni Taisuru Kotai Ryoho -RANKL Kotai (denosumab)' JOURNAL OF CLINICAL AND EXPERIMENTAL MEDICINE vol. 219, no. 1, 2006, pages 91 - 96, XP008171092
- YOSHIYA TOMIMORI ET AL.: 'Hakotsu Saibo Bunka Inshi RANKL Oyobi RANKL Chuwa Kotai no in vivo eno Oyo' BIO CLINICA vol. 25, no. 1, 10 January 2010, pages 70 - 74, XP008171259
- TAISHIN AKIYAMA ET AL.: 'RANKL Signal ni yoru Men'eki Hassei to Men'eki Kan'yo no Seigyo' EXPERIMENTAL MEDICINE vol. 27, no. 6, 2009, pages 865 - 870, XP008171260
- TAISHIN AKIYAMA: 'Kyosen Izonteki na Jiko Men'eki Kan'yo Yudo no Bunshi Kiban' MINOPHAGEN MEDICAL REVIEW vol. 54, no. 3, 2009, pages 155 - 165, XP008171091

## Description

### Technical field

The present invention relates to a composition for potentiating anti-cancer immunity.

### Background Art

### 1. Anti-cancer immune response of T lymphocyte and problem therewith

Methods for treating cancer by inducing an immune response against cancer have been under development recently. One such method uses an immune response of T lymphocytes that recognize an antigen (a cancer-related antigen) expressed in cancer. The method is problematic in that a cancer-related antigen is originally a protein (autoantigen) that is self-expressed.

Immune responses against autoantigens are harmful to individual subjects. Hence, humans are provided with a mechanism for suppressing the harmful effects thereof. The efficiency of an immune response against a cancer-related antigen regarded as an autoantigen is thought to decrease due to the same mechanism.

One mechanism for suppressing immune responses against autoantigens is a mechanism that removes T lymphocytes capable of responding to autoantigens during the development thereof. Most T lymphocytes differentiate in the thymus gland. At such time, T lymphocytes that recognize autoantigens are induced to undergo cell death during differentiation and/or maturation. As a result, such T lymphocytes are removed before their immune response.

One type of cells required for the removal mechanism is a medullary thymic epithelial cell (mTEC). Medullary thymic epithelial cells are among the epithelial cells that form the thymus gland structure, but they have features unseen among other epithelial cells. Specifically, medullary thymic epithelial cells express ectopically an autoantigen group that is generally expressed specifically in peripheral tissue, so as to remove T lymphocytes that recognize the autoantigens (Non-patent document 1).

It has also been found that medullary thymic epithelial cells express a cancer-related antigen as an autoantigen (Non-patent document 2). Specifically, it has been inferred that medullary thymic epithelial cells remove T cells capable of responding to cancer within the thymus gland, thus rather functioning against an immune response of an individual to cancer. However, there is no method for suppressing the functions of medullary thymic epithelial cells in an adult subject. Thus it is unclear whether the suppression of the functions of medullary thymic epithelial cells results in suppressing an increase in cancer.

### 2. RANKL (Receptor activator of NF-κB ligand) bioactivity and application thereof to treatment of disease

RANKL is a cytokine belonging to the TNF family, which induces cell differentiation and the like by binding to RANK, which is the receptor thereof.. Regarding RANKL, the necessity of RANKL in bone resorption in particular is well-known as an essential factor for osteoclast differentiation. A neutralizing antibody that suppresses the functions of RANKL is being used as a therapeutic agent for human osteoporosis (Non-patent document 3). It has also been reported that RANKL is involved in differentiation and/or maturation of mammary epithelial cells and is important for the initial development stage of mammary epithelial cell-derived breast cancer (Non-patent document 4).

Meanwhile, the present inventors have revealed that medullary thymic epithelial cells differentiate through the action of RANKL (receptor activator of NF-κB ligand) (Non-patent document 5), and the differentiation-inducing activity of medullary thymic epithelial cells resulting from the action of RANKL requires TRAF6 (TNF receptor associated factor 6), which is an intracellular signal transduction factor (Non-patent document 6).

Moreover, an attempt to suppress osteoclasts important for cancer bone metastasis and thus to suppress cancer bone metastasis has been conducted through blocking the RANKL action (Non-patent document 7).

### Citation List

### Non-patent documents

Non-patent document 1: Kyewski B et al.; Annu Rev Immunol. 2006; 24: 571-606.
Non-patent document 2: Gotter J et al.; J Exp Med. 2004; 199: 155-66.
Non-patent document 3: Stolina M et al.; Adv Exp Med Biol. 2007; 602: 143-50.
Non-patent document 4: Gonzalez-Suarez E et al.; Nature. 2010; 468: 103-7.
Non-patent document 5: Akiyama T et al.; Immunity. 2008 Sep 19; 29 (3): 423-37.
Non-patent document 6: Akiyama T et al.; Science. 2005; 308: 248-51.
Non-patent document 7: Dougall WC et al.; Curr Opin Support Palliat Care. 2007; 1: 317-22.

### Summary of the Invention

An object of the present invention is to provide a cancer immunopotentiating agent containing a compound that potentiates an immune response to cancer, by blocking the action of RANKL in an adult subject, suppressing an increase of cacner, and blocking the RANKL action as defined in Claims 1-4.

It has been reported that RANKL directly acts on breast cancer epithelial cells, so as to induce the development of breast cancer and that a RANK-Fc protein suppressing the RANKL action suppresses the development of breast cancer (Gonzalez-Suarez E et al.; Nature. 2010; 468: 103-7). However, these effects are suggested to be exhibited at the point of action at which normal cells are transformed into cancer, although they do not suppress the proliferation of already cacerous cells. Moreover, RANKL directly acts on breast cancer epithelial cells, and no immune response to cancer is involved therewith.

Moreover, an attempt has been conducted to suppress osteoclasts important for cancer bone metastasis through blocking of the RANKL action, and thus to suppress cancer bone metastasis (Dougall WC et al.; Curr Opin Support Palliat Care. 2007; 1: 317-22). However, the action is also problematic in that it is unrelated to the induction of an immune response of T lymphocytes and cannot be applied to cancer that does not undergo bone metastasis.

The present inventors have discovered that through suppression of the RANKL action or the signal transduction mechanism thereof, factors required for or involved in survival, maintenance, and functions of medullary thymic epithelial cells can be suppressed or inhibited. Through the action, cancer-responsive T lymphocytes that are generally removed by cell death are caused to survive in the thymus gland. As a result, the capacity of an individual to achieve an anti-cancer immune response can be enhanced and an increase in cacner can be suppressed. Regarding the action, RANKL is not required to directly act on cancer. Therefore, RANKL is applicable to cancer treatment that does not directly act on cancer.

The present inventors have conducted further intensive investigations. Thus, they have examined whether or not the proliferation of medullary thymic epithelial cells decreases, an anti-cancer immune response is potentiated, and an increase in cancer is suppressed, through blocking of the RANKL action. Specifically, the present inventors have verified that the functions of medullary thymic epithelial cells can be suppressed by neutralizing and suppressing the RANKL action using an antibody against RANKL. A RANKL neutralizing antibody was subcutaneously administered to 6- to 8-week-old mice with sufficiently mature thymus glands. Two (2) weeks later, the thymus glands were analyzed. As a result, in a group to which the RANKL neutralizing antibody had been administered, significant decreases in the number of mature medullary thymic epithelial cells were confirmed using a flow cytometer and immunohistochemical staining. Furthermore, a significant decrease in the expression level of a gene group controlling the functions of medullary thymic epithelial cells was also confirmed. These results indicate that the functions of medullary thymic epithelial cells can be effectively suppressed by administration of the RANKL neutralizing antibody. Moreover, the administration of the RANKL neutralizing antibody suppressed an increase in cancer to a significantly higher degree than the administration of a control antibody. Furthermore, the average survival date of mice to which the RANKL neutralizing antibody had been administered increased.

As described above, the present inventors have discovered that through suppression of the RANKL action via administration of the RANKL neutralizing antibody to an adult subject, the survival, maintenance, and functions of medullary thymic epithelial cells of the adult subject can be inhibited, and an increase of cancer can be suppressed. Thus, they have completed the present invention.

Specifically, the present invention is as follows.
[1] A cancer immunopotentiating agent, containing a RANKL antagonist as an active ingredient as defined in Claims 1-4
[2] The cancer immunopotentiating agent of [1], wherein the RANKL antagonist is an anti-RANKL neutralizing antibody.
[3] The cancer immunopotentiating agent of [2], wherein the anti-RANKL neutralizing antibody is clone OYC1.
[4] The cancer immunopotentiating agent of any one of [1] to [3], which potentiates an anti-cancer immune response of a T lymphocyte.
[5] A cancer immunotherapeutic agent, containing the cancer immunopotentiating agent of any one of [1] to [4].

The composition of the present disclosure containing a RANKL antagonist as an active ingredient can potentiate anti-cancer immunity *in vivo* and prevent carcinogenesis and cancer progression.

The description includes the contents described in the description and/or drawings of JP Patent Application No. 2011-079167 based on which the priority of the present application is claimed.

### Brief Description of the Drawings

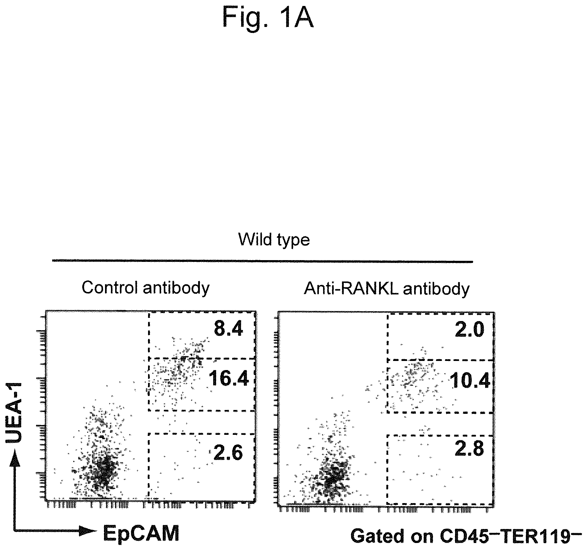

Fig. 1A shows the results of analyzing medullary thymic epithelial cells (RANKL neutralizing antibody; N = 3, control; N = 2) using a flow cytometer (No. 1).

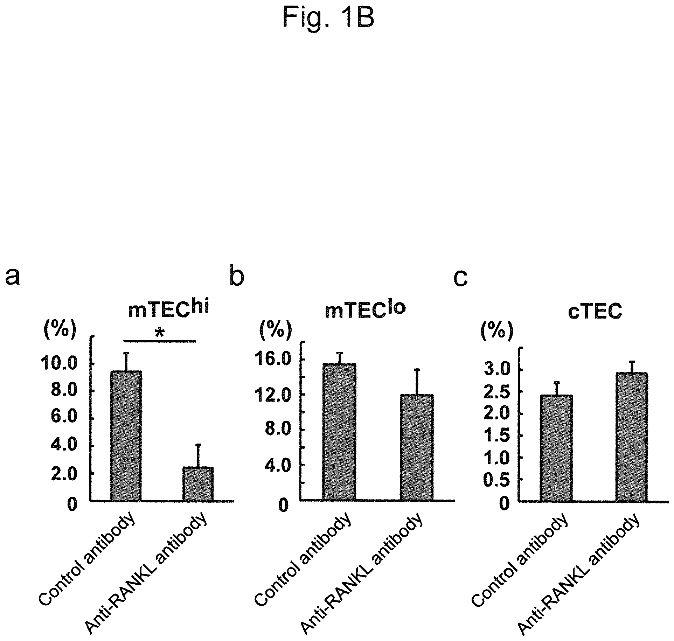

Fig. 1B shows the results of analyzing medullary thymic epithelial cells using a flow cytometer (RANKL neutralizing antibody; N = 3, control; N = 2) (No. 2). In Fig. 1B, "a" denotes mTEChi, "b" denotes mTEClo, and "c" denotes cTEC. In this figure, an asterisk indicates that a significant difference was observed as a result of a significance difference test.

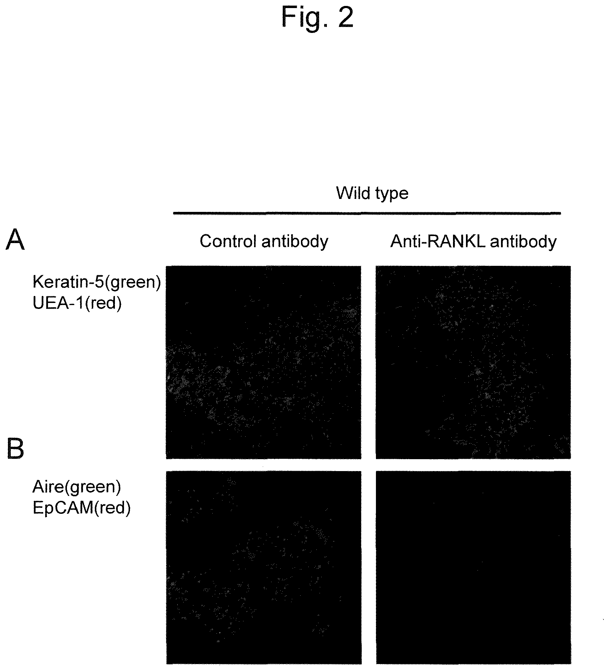

Fig. 2 shows the results of analyzing (N = 3) medullary thymic epithelial cells by immunohistochemical staining. Fig. 2A shows the presence of Keratin-5 (green) and UEA-1 (red) (mature medullary thymic epithelial cell marker). Fig. 2B shows the presence of Aire (green) (a functional molecule of medullary epithelial cells) and EpCAM (red) (medullary epithelial cell marker).

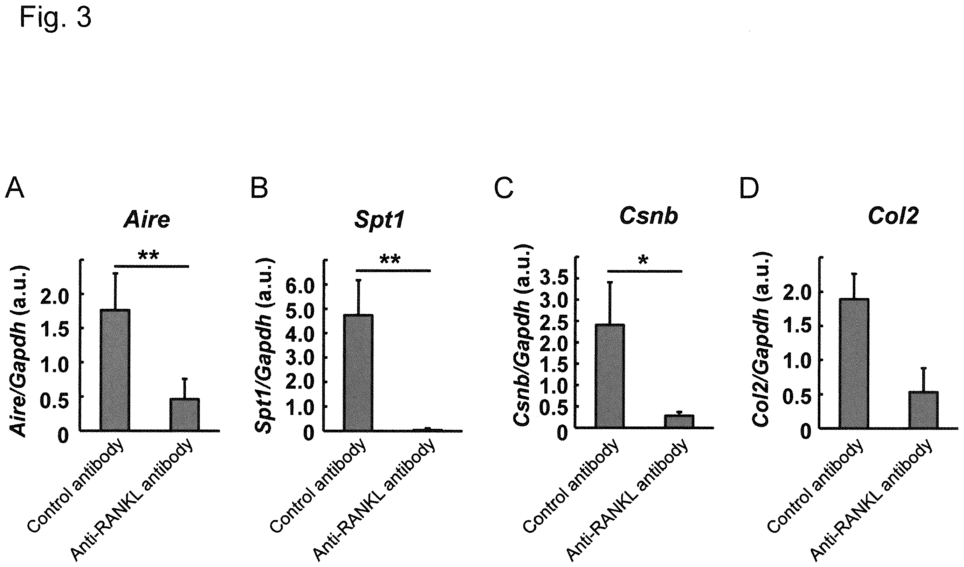

Fig. 3 shows the results of analyzing (N = 3) functional factors of medullary thymic epithelial cells by real time PCR. Fig. 3A, B, C, and D show the results of Aire, Spt1, Csnb, and Col2, respectively. In this figure, an asterisk indicates that a significant difference was observed as a result of a significance difference test.

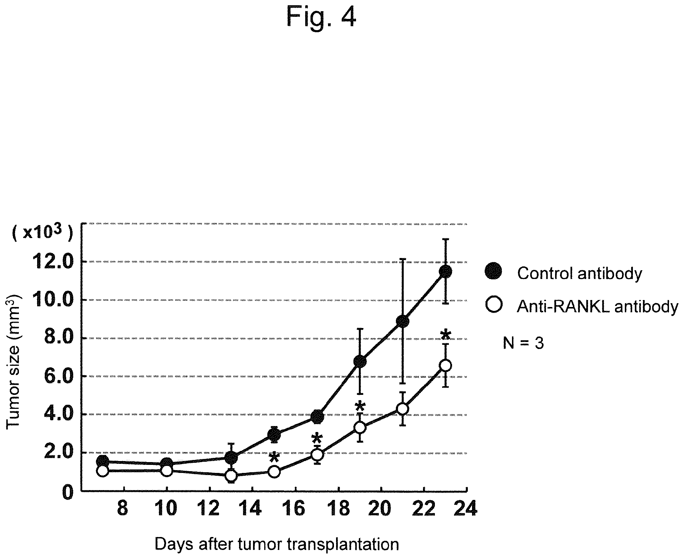

Fig. 4 shows the results of an experiment of cancer transplantation into mice treated with the RANKL neutralizing antibody. In this figure, an asterisk indicates that a significant difference was observed as a result of a significance difference test.

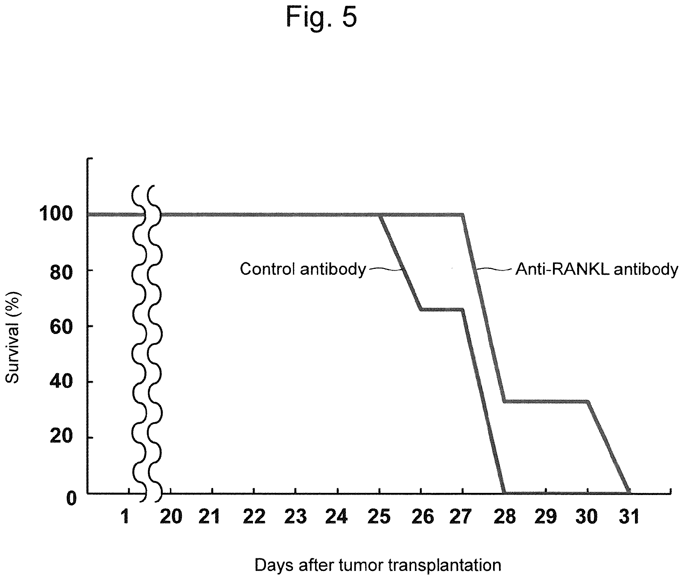

Fig. 5 shows survival curves after cancer transplantation into mice treated with the RANKL neutralizing antibody.

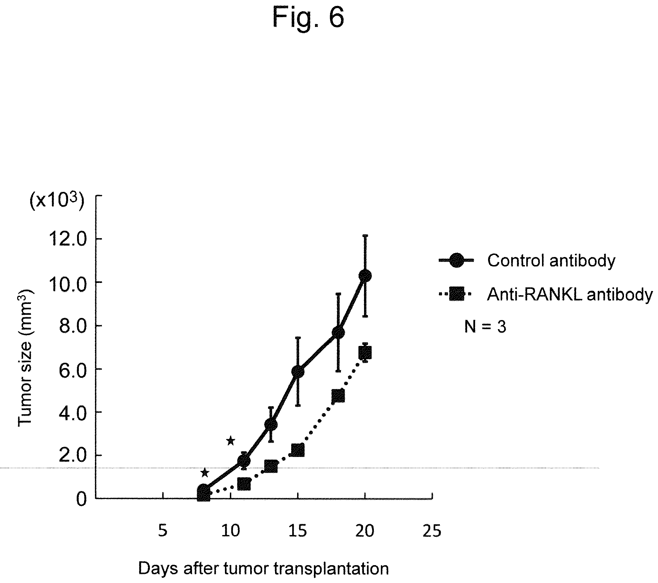

Fig. 6 shows the effects of potentiating cancer immunity of thymic lymphocytes of mice to which the RANKL neutralizing antibody had been administered, which are represented by changes in tumor size. In this figure, an asterisk indicates that a significant difference was observed as a result of a significance difference test.

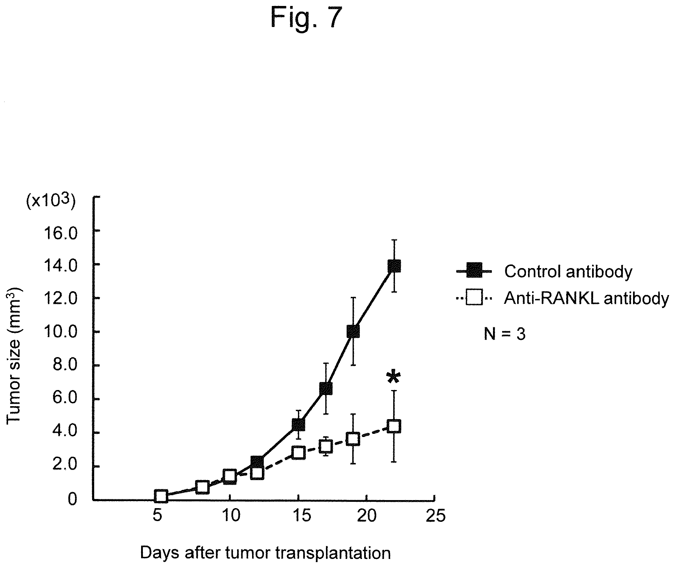

Fig. 7 shows the effects of potentiating cancer immunity of spleen lymphocytes of mice to which the RANKL neutralizing antibody had been administered, which are represented by changes in tumor size. In this figure, an asterisk indicates that a significant difference was observed as a result of a significance difference test.

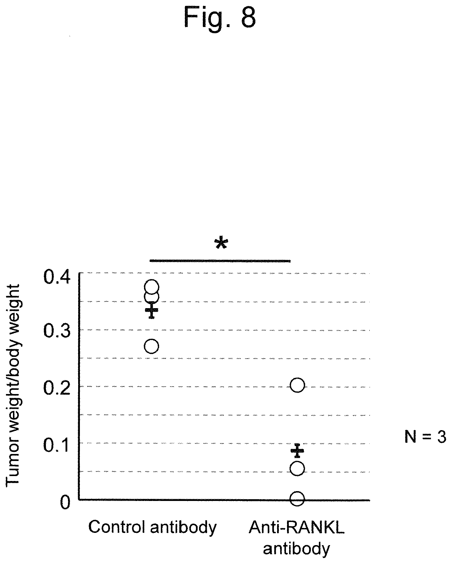

Fig. 8 shows the effects of potentiating cancer immunity of spleen lymphocytes of mice to which the RANKL neutralizing antibody had been administered, which are represented by tumor weight per body weight. In this figure, an asterisk indicates that a significant difference was observed as a result of a significance difference test.

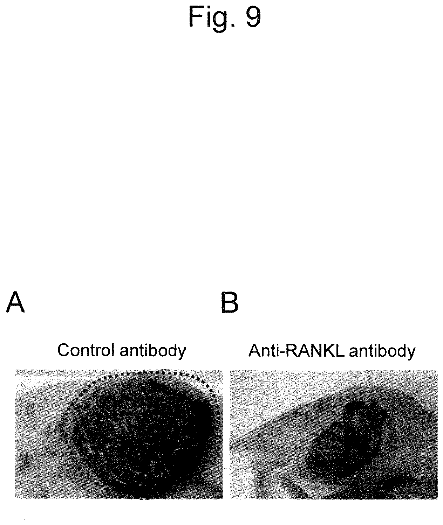

Fig. 9 shows photographs showing increases in cancer in cancer-cell-transplanted mice to which the RANKL neutralizing antibody (anti-RANKL antibody) or the control IgG had been administered.

### Embodiments for Carrying out the Invention

The present invention will be described more specifically below.

RANKL (Receptor activator of NF-κB ligand) is a ligand of RANK (NF-κB receptor activator), which is a member of the TNF super family, and is a type 2 transmembrane protein having an intracellular domain (the domain consisting of amino acid 1 to amino acid 48 from the N terminus of RANKL), a transmembrane domain, and an extracellular domain (JP Patent Publication (Kohyo) No. 2002-509430 A, JP Patent Publication No. 3523650).

The present invention is a composition containing a RANKL antagonist as defined in Claims 1-4 capable of specifically inhibiting RANKL action as an active ingredient. The RANKL antagonist inhibits RANKL action and suppresses the proliferation and differentiation of medullary thymic epithelial cells (mTEC), so as to inhibit the functions thereof. As a result, an immune response of an individual subject to cancer increases.

An example of a RANKL antagonist to be used in the present invention is a substance that binds to RANKL, so as to stop RANKL from binding to its receptor, RANK. An example of such a substance is an anti-RANKL neutralizing antibody that neutralizes RANKL and thus inhibits the RANKL action. Such a neutralizing antibody is also referred to as an anti-RANKL antagonist antibody. The anti-RANKL neutralizing antibody preferably binds to an extracellular domain of RANKL. The animal species from which RANKL (which an antibody of the present invention recognizes and to which it binds) is derived is not limited. RANKL is preferably human RANKL (hRANKL) or murine RANKL (mRANKL), and is further preferably hRANKL. Also, examples of a RANKL antagonist include osteoprotegerin (OPG), known as a RANKL decoy receptor, soluble-type RANK comprising an extracellular domain of RANK, a fusion protein prepared with such a domain and an Fc region of IgG, and a fragment thereof, which binds to RANKL to stop RANKL from binding to its receptor, RANK. Further examples of a RANKL antagonist include a compound having a structure analogous to that of RANKL, a compound having a structure analogous to that of OPG, and a compound having a structure analogous to that of soluble-type RANK, which binds to RANKL to stop RANKL from binding to its receptor, RANK.

Examples of the anti-RANKL neutralizing antibody of the present invention include anti-mouse RANKL antibodies such as clone OYC1 (Oriental Yeast Co., ltd. Catalog No. 47104001) and anti-human RANKL antibodies such as clone 7H12-4G (Oriental Yeast Co., ltd. Catalog No. 47102000). These anti-RANKL antibodies are available from Oriental Yeast Co., ltd.

The anti-RANKL antibody of the present invention can be obtained as a polyclonal antibody or a monoclonal antibody by a known method, and is preferably a monoclonal antibody. Examples of a monoclonal antibody include a monoclonal antibody that is produced by a hybridoma, and a monoclonal antibody that is produced by a host transformed by a genetic engineering technique using an expression vector containing an antibody gene. Such a monoclonal antibody-producing hybridoma can be prepared by a known technique as described below. Specifically, monoclonal antibody-producing cells can be prepared by performing immunization by a known immunization method using a membrane-type or soluble-type RANKL or a fragment peptide thereof as a sensitizing antigen, fusing the thus obtained immune cells to known parent cells by a general cell fusion method, and then screening for cells producing a monoclonal antibody by a known screening method. As RANKL to be used for immunization, an extracellular domain or a fragment of mouse-derived RANKL or human-derived RANKL may be used. Upon immunization with RANKL, it may be bound to a carrier protein such as bovine serum albumin (BSA) or keyhole limpet hemocyanin, and then used. Furthermore, a recombinant monoclonal antibody that can be used herein is prepared by cloning an antibody gene from a hybridoma, incorporating it into an appropriate vector, introducing the vector into a host, and then producing it by gene recombination techniques (e.g., see Vandamme, A. M. et al., Eur. J. Biochem. 1990; 192: 767-775). At this time, DNA encoding an antibody heavy chain (H chain) and DNA encoding an antibody light chain (L chain) are separately incorporated into expression vectors, following which host cells may be transformed simultaneously with the vectors. Alternatively, DNA encoding an H chain and a L chain may be incorporated into a single expression vector, following which a host cell can be transformed with the vector (International Patent Publication WO 94/11523 pamphlet). Moreover, with the use of a transgenic animal, a recombinant antibody can also be produced. For example, an antibody gene is inserted in the middle of a gene encoding a protein (e.g., goat β casein) to be inherently produced in milk, and thus a recombinant antibody gene is prepared as a fusion gene. A DNA fragment containing the fusion gene in which the antibody gene has been inserted is injected into a goat embryo, and then the embryo is introduced into a female goat. A desired antibody can be obtained from milk produced by transgenic goats born from the goat that has received the embryo, or progenies thereof (Ebert, K.M. et al., Bio/Technology 1994; 12: 699-702).

The thus obtained antibody inhibits the RANKL action. Whether or not this inhibits the proliferation and differentiation of medullary thymic epithelial cells can be confirmed by a method using mice as described in examples below, for example.

Further examples of the anti-RANKL antibody of the present invention include gene recombinant antibodies prepared by artificial modification for the purpose of decreasing heterologous antigenicity against humans, such as a chimeric antibody, a humanized antibody, and a human antibody. All of these antibodies can be produced by known methods. A chimeric antibody can be obtained by obtaining DNA encoding an antibody V region, linking the DNA and DNA encoding a human antibody C region, incorporating the resultant to an expression vector, introducing the vector into a host, and then causing the host to produce the chimeric antibody. A humanized antibody is prepared by transplanting the complementarity determining region (CDR) of a non-human mammalian antibody such as a mouse antibody into the complementarity determining region (CDR) of a human antibody. Thus, such a humanized antibody has a non-human animal antibody-derived CDR and a human antibody-derived framework region. A humanized antibody can be prepared by a known method (see European Patent Application Publication No. EP 125023 and WO 96/02576). A humanized antibody may also be referred to as a reshaped human antibody. For a chimeric antibody C region and a humanized antibody C region, portions of a human antibody are used. For example, Cγ1, Cγ2, Cγ3, and Cγ4 can be used in the H chain and Cκ and Cλ can be used in the L chain. Moreover, a human antibody C region may be modified to improve the stability of the antibody or the production thereof.

A human antibody can be obtained by introducing a human antibody gene locus, and then administering an antigen to a transgenic animal capable of producing a human-derived antibody, for example. An example of such a transgenic animal is a mouse. Methods for producing mice that can produce a human antibody are as described in: U.S. Patent No. 7,145,056 (specification) (Xeno Mouse (registered trademark)); U.S. Patent No. 5,612,205 (specification), U.S. Patent No. 5,981,175 (specification), U.S. Patent No. 5,814,318 (specification), U.S. Patent No. 5,545,806 (specification) (HuMAb-Mouse (registered trademark)); Tomizuka, K. et al., Nature Genet., 16, 133-143, 1997 (TransChromo Mouse (trademark)); and Ishida I. et al., Cloning and Stem Cells, 4, 85-95, 2002 (KM Mouse (trademark)), for example. A human antibody can also be prepared by a phage display method using a phage that displays a human antibody fragment on the surface. Specifically, human antibody heavy chain and light chain are obtained from human B cells, an artificial sequence is added to the CDR region, a library of phages expressing a human variable region is prepared by a phage display method, and then a human antibody capable of binding to a target as desired is selected. Preparation of a human antibody by a phage display method is as described in International Patent Publication WO 1992/015679 pamphlet, or the like.

Examples of an anti-RANKL antibody include not only a complete antibody, but also a functional fragment thereof. The term "functional fragment" of an antibody refers to a portion (partial fragment) of an antibody, having at least one effect of the antibody on an antigen. Specific examples of a functional fragment include F (ab')₂, Fab', Fab, Fv, disulfide-bond Fv, single-chain Fv (scFv), and polymers thereof [D. J. King., Applications and Engineering of Monoclonal Antibodies., 1998 T. J. International Ltd].

When a monoclonal antibody is used, only one type of monoclonal antibody may be used or two or more types of monoclonal antibody that recognize different epitopes may also be used.

The composition of the present disclosure is a cancer immunopotentiating agent, a cancer immunity activator, or an agent for accelerating potentiation of cancer immunity that contains the above anti-RANKL antibody as an active ingredient. The composition can increase cancer immune reaction *in vivo*, inhibit the onset or progression of cancer, and exhibit therapeutic or preventative effects on cancer. Specifically, the composition can be used as a cancer immunotherapeutic agent.

The anti-RANKL antibody suppresses the functions of medullary thymic epithelial cells. Medullary thymic epithelial cells serve to remove T lymphocytes that recognize an autoantigen. Therefore, medullary thymic epithelial cells eliminate T lymphocytes against a cancer-related antigen that is formed autologously *in vivo*. The anti-RANKL antibody suppresses the functions of medullary thymic epithelial cells, so that T lymphocytes against a cancer-related antigen are not eliminated, resulting in an immune reaction to cancer. Specifically, the anti-RANKL antibody potentiates an anti-cancer immune response by T lymphocytes, so as to potentiate cancer immunity through the action of T lymphocytes, and thus exhibits therapeutic or preventative effects on cancer without directly acting on cancer cells. Accordingly, a composition containing the anti-RANKL antibody can be used as a cancer immunopotentiating agent that potentiates and increases an anti-cancer immune response by T lymphocytes. T lymphocytes, the anti-cancer immune response of which is increased by the anti-RANKL antibody, migrate to and are then localized at secondary lymphatic tissue such as the spleen after the development thereof in the thymus gland. T lymphocytes localized in the secondary lymphatic tissue efficiently cause an immune reaction to cancer.

The dosage differs depending on symptoms, age, body weight, and the like. Generally in the case of peroral administration, the amount of an antibody to be administered to an adult ranges from about 0.01 mg to 1000 mg per day, and the antibody may be administered once or several times a day. Moreover, in the case of parenteral administration, the amount of an antibody ranging from about 0.01 mg to 1000 mg per day may be administered via subcutaneous injection, intramuscular injection, or intravenous injection.

The composition contains a carrier, a diluent, and an excipient that are generally used in the field of preparations. For example, as carriers and excipients for tablets, lactose, magnesium stearate, and the like are used. As an aqueous solution for injection, saline, an isotonic solution containing dextrose or other adjuvants, or the like is used. This may be used in combination with an appropriate solubilizing agent, such as alcohol, polyalcohol (e.g., propylene glycol), or a nonionic surfactant. As oily fluids, sesame oil, soybean oil, and the like are used. As solubilizing agents, benzyl benzoate, benzilalcohol, and the like may also be used in combination.

Examples of cancer to be treated or prevented with the use of the composition of the present invention include, but are not limited to, gastric cancer, lung cancer, liver cancer, cancer of the colon, anal·rectal cancer, esophageal cancer, pancreatic cancer, breast cancer, renal cancer, skin cancer, uterine cancer, prostate cancer, bladder cancer, adrenal cancer, brain/nervous system tumor, leukemia, lymphoma, and mesoepithelioma.

T lymphocytes in a cancer patient to which the composition of the present invention containing the anti-RANKL antibody has been administered have potentiated anti-cancer immunity activity due to the action of the composition. Such T lymphocytes are further increased and then can be used for treatment of cancer. Specifically, the composition of the present invention containing the anti-RANKL antibody is administered to a cancer patient, the composition and an existing immune activation therapy are used in combination, and thus the number of T lymphocytes against cancer *in vivo* in the cancer patient is increased. Lymphocytes are collected from the patient after administration, so as to increase lymphocytes *in vitro*, and then the lymphocytes may be administered to the patient.

The present invention will be more specifically described by way of Examples below. The present invention should not be limited to the following Examples.

### Example 1 Effect of RANKL neutralizing antibody on medullary thymic epithelial cell

### (1) Method for administration to mice

Clone OYC1 (Oriental Yeast Co., ltd. Catalog No. 47104001) of a RANKL neutralizing antibody (3 cases) and clone OYC2 (Oriental Yeast Co., ltd. Catalog No. 47103001) of a mouse RANKL antibody (control antibody) (2 cases) having no capacity for neutralization were subcutaneously administered under Nembutal anesthesia to CD40-deficient or wild-type (C57BL/6JJc1, Clea Japan Inc.) 6-week-old female mice at a dose of 5 mg/kg. Two weeks later, the thymus glands were excised from the mice. Expression analyses were conducted using (2) a flow cytometer, (3) immunostaining of frozen sections, and (4) Real Time PCR method.

### (2) Flow cytometric analysis

Several cuts were made with scissors on each portion (about 20 mg) of the thymus glands in a petri dish containing 1 ml of PBS. The resultant was transferred to a 1.5-ml tube containing 1 ml of RPMI-1640 medium and then slowly stirred with a rotator for 10 minutes at 4°C. The supernatant was collected together with the above PBS into another 15-ml tube. RPMI-1640 medium (1 ml) containing 0.125% Collagenase/Dispase (Roshe) and 0.01% DNaseI was added, followed by 15 minutes of incubation at 37°C. During incubation, pipetting was performed every 5 minutes. The supernatant was transferred to the 15-ml tube, and then new RPMI-1640 medium containing 0.125% Collagenase/Dispase and 0.01% DNaseI was added. This procedure was repeated 4 times. A cell population in sufficiently disintegrated thymus gland was obtained. 1 x 10⁷ cells were suspended in 100 µl of FACS buffer (2% FCS-containing PBS), and then blocking treatment was performed with an anti-CD16/32 antibody on ice for 20 minutes. Staining was performed with a labeled anti-CD45 antibody, an anti-TER-119 antibody, an anti-EpCAM antibody, and UEA-1 lectin on ice for 20 minutes. After washing twice with FACS buffer, FACS analysis was conducted (Fig. 1). Upon analysis, CD45⁻ thymocytes and TER119⁻ thymocytes were stained with EpCAM (thymic epithelial cell marker) and UEA-1 (medullary thymic epithelial cell marker). A cell fraction (mTEChi) (of mature medullary epithelial cells as a result of differentiation) positive for EpCAM and positive for UEA-1 at a high level, a cell fraction (mTEClo) (of medullary epithelial cells) positive for EpCAM and positive for UEA-1 at a low level, and a cell fraction (cTEC) (of cortical epithelial cells) positive for EpCAM and negative for UEA-1 were separately analyzed. Fig. 1A shows the results of analysis using a flow cytometer. Fig. 1B shows graphs summarizing analytical results. As shown in Fig. 1, mature medullary epithelial cells were found to significantly decrease in the group to which the RANKL neutralizing antibody had been administered.

### (3) Immunostaining of frozen section

A portion (about 40 mg) of thymus glands was frozen in an OCT compound with liquid nitrogen, and then a frozen section sample with a thickness of about 5 µm was prepared on slide glass. After acetone fixation, the resultant was washed with PBS, subjected to blocking treatment with a PBS solution containing anti-goat antibody (10%) at room temperature for 20 minutes, and then treated with an anti-Keratin-5 antibody and an anti-UEA-1 antibody, or an anti-EpCAM antibody and an anti-Aire antibody at room temperature for 1 hour. After washing with PBS, the resultants were treated with fluorescence (Alexa488 and Alexa546)-labeled secondary antibody at room temperature for 40 minutes. After washing with PBS, fluorescent images were taken using a confocal microscope (Zeiss 710) (Fig. 2). Fig. 2A shows the results for Keratin-5 and UEA-1, wherein Keratin-5 is indicated with green fluorescence and UEA-1 is indicated with red fluorescence (mature medullary thymic epithelial cell marker). Portions with red fluorescence were conspicuously observed for the group to which the control antibody had been administered; however, for the group to which the RANKL neutralizing antibody had been administered, only green fluorescence was observed and almost no red fluorescence was observed. Fig. 2B shows the results for Aire (a functional molecule of medullary epithelial cells) and EpCAM (medullary epithelial cell marker), wherein Aire is indicated with green fluorescence and EpCAM is indicated with red fluorescence. For the group to which the control antibody had been administered, portions with green fluorescence and portions with red fluorescence were observed to be dominant; however, for the group to which the RANKL neutralizing antibody had been administered, portions with green fluorescence and portions with red fluorescence were observed to drastically decrease. The results indicate that for the group to which the RANKL neutralizing antibody had been administered, mature medullary epithelial cells had significantly decreased.

### (4) Expression analysis on functional gene of medullary thymic epithelial cell using quantitative Real Time PCR method

RNA was collected using a TRIZOL (registered trademark) reagent (Invitrogen) from a portion (about 10 mg) of the thymus gland cryopreserved at -80°C. RNA was treated with DNaseI at 37°C for 30 minutes, so as to remove contaminating DNA therein. cDNA was prepared by a reverse transcriptase reaction using about 2 µg of RNA as a template and Random Hexamer as a primer. Gene expression of Aire, Spt1, Csnb, and Col2 was analyzed by Real Time PCR (Applied Biosystems) using the cDNA as a template (Fig. 3). Expression of "functional factor(s)" of mature medullary thymic epithelial cells was verified by quantitative RT-PCR. The results of these analyses are all represented by values relative to the expression level of GAPDH. Fig. 3A, B, C, and D indicate the results for Aire, Spt1, Csnb, and Col2, respectively. As shown in Fig. 3, the expression levels of factors (e.g., Aire and tissue-specific antigen) required for the functions of mature medullary thymic epithelial cells were found to significantly decrease in the group to which the neutralizing antibody had been administered.

### (5) Effect of RANKL neutralizing antibody on increase in cancer cell

The RANKL neutralizing antibody (Oriental Yeast Co., ltd., clone, OYC1) and the control antibody (Sigma-Aldrich, rat purified IgG) (3 cases each) were subcutaneously administered via the cervix under Nembutal anesthesia to wild-type (C57BL/6JJc1, Clea Japan Inc.) 6-week-old female mice at a dose of 5 mg/kg. Two weeks later, second administration was performed with the same dosage. A further two weeks later, cultured lymphoma EL-4 (1×10⁶ cells) was transplanted subcutaneously to the right flank of each mouse under Nembutal anesthesia. Tumor size measurement was started on day 5 after transplantation, and then the survival date thereafter was examined.

Tumor size was found by measuring the short diameter ("a" mm) and the long diameter ("b" mm) by electronic vernier calipers, thereby finding the volume by (a×b)^{3/2}×π/6. Survival % was found by the following formula (the number of mice that had survived/the number of mice tested (N = 3) x 100) (Fig. 4). In an experiment, the results of which are shown in Fig. 4, the RANKL neutralizing antibody (open circle) or the control antibody (shaded circle) was administered twice every two weeks to each wild-type mouse. Two weeks later, mouse cancer cells EL4 were subcutaneously transplanted. As shown in Fig. 4, increases in cancer were significantly suppressed in the group of mice to which the RANKL neutralizing antibody had been administered, unlike the mice to which the control antibody had been administered.

Survival of each mouse subjected to transplantation was confirmed every day (Fig. 5). In an experiment, the results of which are shown in Fig. 5, the RANKL neutralizing antibody or the control antibody was administered twice every two weeks to each wild-type mouse. Two weeks later, mouse cancer cells EL4 were subcutaneously transplanted, and then the survival of the mice was confirmed. Fig. 5 shows survival curves for the group of mice to which the RANKL neutralizing antibody had been administered and the group of mice to which the control antibody had been administered. As shown in Fig. 5, survival % for the group of mice to which the RANKL neutralizing antibody had been administered exhibited significant improvement compared with the group of mice to which the control antibody had been administered.

### Example 2 Effect of potentiating cancer immunity of RANKL neutralizing antibody via lymphocyte

To examine if lymphocytes of a mouse to which the RANKL neutralizing antibody had been administered had an effect of suppressing an increase in cancer, thymic lymphocytes or spleen lymphocytes of a mouse to which the RANKL neutralizing antibody had been administered were transplanted into a nude mouse, and then the effect of suppressing cancer was examined.

The RANKL neutralizing antibody (Oriental Yeast Co., ltd., clone, OYC1) and the control antibody (Sigma-Aldrich, rat purified IgG) (3 cases each) were subcutaneously administered via the cervix under Nembutal anesthesia to wild-type (Balb/cA JCL, Clea Japan Inc.) 6-week-old female mice at a dose of 5 mg/kg. Two weeks later, second administration was performed with the same dosage. Two weeks after the final administration, lymphocytes were collected from the thymus gland or the spleen. Lymphocytes were intravenously injected simultaneously with the subcutaneous transplantation of a cancer cell line (Meth A; 5 x 10⁶ cells) into 6-week-old female BALB/cA nu/nu mice (Clea Japan Inc.).

Tumor size was found by measuring the short diameter ("a" mm) and the long diameter ("b" mm) by electronic vernier calipers, thereby finding the volume by the formula of: (a×b)^{3/2}×π/6 (Fig. 6 and Fig. 7). In an experiment, the results of which are shown in Fig. 6, the RANKL neutralizing antibody or the control antibody was administered twice every two weeks to each wild-type mouse (Balb/cA JCL, Clea Japan Inc.). A further two weeks later, thymic lymphocytes were collected. Lymphocytes were intravenously injected simultaneously with subcutaneous transplantation of a cancer cell line (Meth A; 5 x 10⁶ cells) into 6-week-old female BALB/cA nu/nu mice (Clea Japan Inc.). After transplantation of cancer cells, tumor size measurement was started on day 8. As shown in Fig. 6, increases in cancer were significantly suppressed in the group (shaded square) of mice to which thymic lymphocytes (derived from a RANKL-neutralizing-antibody-administered mouse) had been injected, compared with the group (shaded circle) of mice to which thymic lymphocytes (derived from a control-antibody-administered mouse) had been injected. In an experiment, the results of which are shown in Fig. 7, Fig. 8, and Fig. 9, the RANKL neutralizing antibody or the control antibody was administered twice every two weeks to each wild-type mouse. Further two weeks later, spleen lymphocytes were collected. Spleen lymphocytes were intravenously injected simultaneously with subcutaneous transplantation of a cancer cell line (Meth A) into 6-week-old female BALB/cA nu/nu mice (Clea Japan Inc.). After transplantation, tumor size measurement was started on day 5. As shown in Fig. 7, increases in cancer were significantly suppressed in the group of mice to which spleen lymphocytes (derived from a RANKL-neutralizing-antibody-administered mouse) had been injected (open square), compared with the group (shaded square) of mice to which spleen lymphocytes (derived from a control-antibody-administered mouse) had been injected. Moreover, in an experiment, the results of which are shown in Fig. 8, cancer was collected on day 22 after transplantation, cancer weights and mouse body weights were measured, and then the ratios thereof were determined. Cancer weights with respect to body weights were significantly lower in the group of mice to which lymphocytes (derived from a RANKL-neutralizing-antibody-administered mouse) had been injected, compared with the group of mice to which lymphocytes (derived from a control-antibody-administered mouse) had been injected. As shown in Fig. 8 and Fig. 9, almost complete remission was observed in one of three cases among the group of mice to which spleen lymphocytes had been injected. Fig. 9A shows an increase in cancer of a mouse to which control IgG was administered. Fig. 9B shows an increase in cancer of a mouse to which the anti-RANKL antibody was administered.

### Industrial Applicability

A RANKL antagonist such as an anti-RANKL neutralizing antibody can be used as a cancer immunotherapeutic agent.

## Claims

1. A RANKL antagonist for use in potentiating immune response to cancer, wherein the RANKL antagonist is an anti-RANKL neutralizing antibody.

2. The RANKL antagonist for use in potentiating immune response to cancer according to claim 1, wherein the anti-RANKL neutralizing antibody is clone OYC1.

3. The RANKL antagonist for use in potentiating immune response to cancer according to any one of claims 1 to 2, which potentiates an anti-cancer immune response of a T lymphocyte.

4. A cancer immunotherapeutic agent, containing the RANKL antagonist for use in potentiating immune response to cancer of any one of claims 1 to 3.

## Patentansprüche

1. RANKL-Antagonist zur Verwendung bei der Verstärkung einer Immunantwort gegen Krebs, wobei der RANKL-Antagonist ein anti-RANKL, neutralisierender Antikörper ist.

2. RANKL-Antagonist zur Verwendung bei der Verstärkung einer Immunantwort gegen Krebs gemäß Anspruch 1, wobei der anti-RANKL, neutralisierende Antikörper der Klon OYC1 ist.

3. RANKL-Antagonist zur Verwendung bei der Verstärkung einer Immunantwort gegen Krebs gemäß irgendeinem der Ansprüche 1 bis 2, der eine anti-Krebs-Immunantwort eines T-Lymphozyten verstärkt.

4. Krebs-Immuntherapeutisches Agens, das den RANKL-Antagonist zur Verwendung bei der Verstärkung einer Immunantwort gegen Krebs gemäß irgendeinem der Ansprüche 1 bis 3 enthält.

## Revendications

1. Antagoniste de RANKL pour une utilisation pour potentialiser une réponse immunitaire à un cancer, dans lequel l'antagoniste de RANKL est un anticorps neutralisant anti-RANKL.

2. Antagoniste de RANKL pour une utilisation pour potentialiser une réponse immunitaire à un cancer selon la revendication 1, dans lequel l'anticorps neutralisant anti-RANKL est le clone OYC1.

3. Antagoniste de RANKL pour une utilisation pour potentialiser une réponse immunitaire à un cancer selon l'une quelconque des revendications 1 à 2, qui potentialise une réponse immunitaire anti-cancer d'un lymphocyte T.

4. Agent immuno-thérapeutique anticancéreux contenant l'antagoniste de RANKL pour une utilisation pour potentialiser une réponse immunitaire à un cancer selon l'une quelconque des revendications 1 à 3.
